# EUROPEAN PATENT APPLICATION

(11) **EP 1 950 293 A1**
(43) Date of publication of application: **30.07.2008**
(21) Application number: 06822051.6
(22) Date of filing: 23.10.2006
(51) Int. Cl.: C12N 15/09, A61K 45/00, A61P 35/00

(54) **METHOD FOR PREDICTION OF EFFECTIVENESS OF RAR- AGONIST**

(30) Priority: 24.10.2005 US 729175 P; 27.06.2006 US 816616 P
(71) Applicant: Taiho Pharmaceutical Co., Ltd., Chiyoda-ku, Tokyo 1018444 (JP)
(72) Inventor: MURAKAMI, Koji, Hanno-shi, Saitama 357-8527 (JP)
(74) Representative: Polypatent
(86) International application number: PCT/JP2006/321031
(87) International publication number: WO 2007/049542

(57) **Abstract**

A method for predicting preventive and/or therapeutic effect of an RAR- α agonist on a malignant tumor, which comprises the steps of measuring an expression level of a class of p160 family molecule and an expression level of a class of SP110 family molecule in a sample collected from the malignant tumor of a patient, and when the class of the p160 family molecule is dominant in a balance between the expression level of the class of the p160 family molecule and the expression level of the class of the SP110 family molecule, determining that an RAR- α agonist is effective for therapeutic treatment of the malignant tumor of the patient.

## Description

### Technical Field

The present invention relates to a method for predicting efficacy of an RAR- α agonist in patients with malignant tumor. More specifically, the present invention relates to a method for conveniently and accurately predicting therapeutic effect of an RAR- α agonist in patients with malignant tumor such as liver cancer.

### Background Art

Liver cancer is highly malignant, and one million patients die of this disease every year. The number of patients is ranked as the fifth in the world [El-Serag H.B. and Mason A.C., Rising incidence of hepatocellular carcinoma in the United States., N. Engl. J. Med., 340, pp.745-750, 1999; Taylor-Robinson S.D., Foster G.R., Arora S., Hargreaves S. and Thomas H.C., Increase in primary liver cancer in the UK, 1979-94, Lancet, 350, pp.1142-1143, 1997].

Liver cancer is thought to be caused by multiple cancer cells that emerge at separate sites simultaneously or sequentially due to exposure of the liver with hepatitis viruses or alcohol [Slaughter DP, Southwick HW., Smejkal W., "Field cancerization" in oral stratified squamous epithelium: Clinical implications of multicentric origin, Cancer, 6, pp.963-968, 1953]. In Japan, it is considered that liver cancer is primarily caused by hepatitis C virus infection. Since hepatitis C virus cannot be eliminated even though cancer cells are temporarily removed or killed, cancer is very likely to recur. The recurrence rate was reported as 16 to 29% over a period of from 1990 to 1997 [Liver cancer, I Changes in number of patients, 3. Counting method of number of patients, Trend of cancer patients and current conditions for therapies, Medical Research, pp.20-30, 2001].

Therapeutic methods of liver cancer include surgical hepatectomy of visible lesions, radiofrequency ablation, ethanol injection therapy, microwave coagulo-necrotic therapy and the like. However, these therapies are sometimes not effective, and chemotherapy as a systemic treatment is desired.
At present, as chemotherapy, intra-arterial injection of CDDP, ADM and 5-FU is performed for the treatment of advanced cancers for which the aforementioned therapies or transcatheter arterial embolization cannot be applied, and cancers recurring after a treatment by applying those therapies. However, it is reported that effectiveness of the intra-arterial injection chemotherapies for the liver using an oily contrast medium, lipiodol, and the aforementioned medicaments in combination is found to be 13.0% of complete response and 30.0% of partial response, and that of chemotherapies in which transcatheter arterial embolization or lipiodol is not used in combination is found to be 2.5% of complete response and 3.1% of partial response, which results of treatment are not fully satisfactory. Moreover, these combination therapies suffer from problems of occurrence of complications such as ulcer and the like [Tanigawa H., Local therapies and intra-arterial injection therapies, Japanese Journal of Cancer Clinics, 40, pp.1490-1497, 1994].

As described above, any standard therapy that ensures stable treatment results to a certain extent for liver cancer has not been established. In particular, the intra-arterial injection therapy has problems from viewpoints of technique and burden on patients, and therefore development of chemotherapy alternative to the therapy has been desired. Further, even a therapeutic treatment for temporarily stabilizing a tumor, i.e., life prolongation without proliferation of tumor cells, is considered not achievable due to high resistance of liver cancer to chemotherapy. Therefore, development of chemotherapy that can achieve tumor preservation has also been strongly desired.

4-[3,5-Bis(trimethylsilyl)benzamido]benzoic acid (henceforth also referred to as "TAC-101" in the specification) is a synthetic retinoid represented by formula (1) shown below. The compound is known to be useful as a therapeutic agent for cancer, cancer cell differentiation inducer, cancer metastasis inhibitor, therapeutic agent for vascular diseases or therapeutic agent for cardiac hypertrophy (Japanese Patent Laid-open Publication (Kokai) No. 2-247185, International Patent Publications WO96/32101 and WO03/089005 and the like).

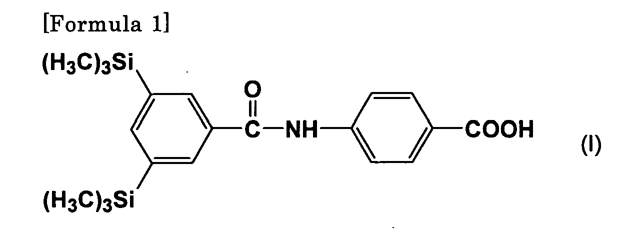

TAC-101 1 is an RAR- α agonist that has an action of binding to the retinoic acid receptor subtype α (henceforth also referred to as "RAR- α "), which is a transcription factor belonging to the nuclear receptor family, to activate transcription via this receptor, and exerts anti-tumor effect by the activation of transcription. It is known that expression of RAR- α increases in patients with liver cancer, (Clin. Cancer Res., 9, pp.3679-3683, 2003), and antitumor activity of TAC-101 was observed in pharmacological studies using an animal model of liver cancer (Clin. Exp. Metastasis. 16, pp.633-643, 1998; Clin. Exp. Metastasis., 16, pp.323-331, 1998; J. Cancer Res., 90, pp.1254-1261, 1999). As for clinical effect of TAC-101 on human liver cancer, 43% of prevention of tumor progression has been found in a phase II study in the United States.

It is known that activation of RAR- α requires binding of a ligand molecule such as retinoic acid. A nuclear receptor activated by binding of a ligand binds to a target DNA sequence to initiate translation of DNA. DNA binds to a histone to form a chromatin structure, and for the activation of transcription, an alteration in the chromatin structure by acetylation of histone is essential. Coactivators that enhance activation of RAR- α transcription are histone acetylases, per se, that are necessary for the acetylation of histone or a class of molecules responsible for recruiting histone acetylases to the transcription activation site. They are basically classified into the class of p300 family molecules which primarily play a role of the former (Curr. Biol., 7(9), pp.689-692, 1997), and the class of p160 family molecules which play a role of the latter (Mol. Endocrinol., 17(9), pp.1681-1692, 2003). These class of coactivator molecules positively regulate the transcription activity. It is known that AIB1 (Amplified In Breast Cancer-1), one of the class of the p160 family molecules, is one of steroid receptor coactivators overexpressed in breast cancer cells, and is useful for diagnosis of steroid hormone-responsive cancer (International Patent Publication WO98/57982). The molecule is also known to be useful for prediction of prostate cancer and prediction of tamoxifen susceptibility of breast cancer (International Patent Publications WO02/10452, WO03/189, WO03/8990 and the like). SRC-1 (Steroid Receptor Coactivator-1) is also one of steroid receptor coactivators and is known to be useful for a therapeutic treatment of diseases associated with steroid receptor activity (International Patent Publication WO97/10337). Furthermore, it is known that TIF2 (Translation Initiation Factor-2) is also a factor that similarly acts on the AF-2 site of a nuclear receptor as a steroid receptor coactivator to promote ligand-dependent transcriptional activation (EMBO J., Jul 15, pp.3667-3675, 1996).

Existence of a class of corepressor molecules that inhibit transcription activity of RAR- α is also known. Many of these molecules have an activity as histone deacetylase and negatively regulate DNA translation via nuclear receptors by promoting chromatinization of histone (White J.H., et al., Vitam. Horm., 68, pp.123-143, 2004). Recently, it has been reported that the class of the SP110 family molecules expressed in hepatocellular carcinoma are RAR- α -selective corepressors (Watashi K., et al, Mol. Cell Biol., 23(21), pp.7498-7509, 2003). It is considered that the transcriptional activation mechanism modulated by nuclear receptors is regulated by balance of these coactivators and corepressors. Further, International Patent Publication WO02/8383 discloses the isolation of the class of SP110 family molecules including SP110b, and they are known to be useful for diagnosis of primary biliary cirrhosis (PBC).

### Disclosure of the Invention

### Problems to be solved by the invention

An object of the present invention is to provide a method for determining efficacy of an RAR-α agonist such as TAC-101 in patients with malignant tumor such as liver cancer. More specifically, the object of the present invention is to provide a method for conveniently and accurately predicting therapeutic effect of an RAR- α agonist on malignant tumor such as liver cancer.

### Means for solving the problem

The inventors of the present invention conducted various researches to achieve the foregoing object, and as a result of the researches, they first found that an RAR-α agonist such as TAC-101 was not effective on a liver cancer without expression of RAR- α, whilst the intensity of the expression of RAR- α did not necessarily correlate with the degree of the transcriptional regulation function, and that there was no clear correlation between the antitumor activity of an RAR- α agonist on liver cancer and the expression of RAR- α. They also found that, among the classes of coactivator molecules, although the class of p160 family molecules such as AIB1, SRC-1 and TIF2 positively correlated with transcription activity of RAR- α induced by an RAR-α agonist, and the expression of SP110b as an RAR-α -selective corepressor negatively correlated with transcription activity of RAR- α induced by an RAR- α agonist, the expression levels of these coactivators and corepressor varied in individual cancer cells, and thus it was difficult to use the levels as an marker for determining efficacy of an RAR- α agonist on liver cancer.

The inventors of the present invention conducted further researches on the basis of these findings. As a result, they found that a ratio of the expression levels of the class of the p160 family molecule as a coactivator and the class of the SP 110 family molecule as a corepressor gave remarkably high correlation with the activation of RAR-α transcription induced by an RAR-α agonist. The present invention was achieved on the basis of the aforementioned findings.

The present invention thus provides a method for predicting therapeutic effect of an RAR- α agonist on a malignant tumor, which comprises the steps of measuring an expression level of a class of p160 family molecule and an expression level of a class of SP110 family molecule in the malignant tumor of a patient and observing a balance between said expression levels.
The present invention also provides a method for predicting therapeutic effect of an RAR- α agonist on a malignant tumor, which comprises the steps of measuring an expression level of a class of p160 family molecule and an expression level of a class of SP110 family molecule in the malignant tumor of a patient, observing a balance between said expression level of the class of the p160 family molecule and the expression level of the class of the SP110 family molecule, and when the class of the p160 family molecule is dominant, determining that an RAR- α agonist is effective for therapeutic treatment of the malignant tumor of the patient.

As preferred embodiments of the aforementioned invention, there are provided the aforementioned method, wherein an expression level of AIB1, SRC-1 or TIF2 among the class of the p160 family molecules is measured; the aforementioned method, wherein an expression level of SP110b as the class of the SP110 family molecules is measured; the aforementioned method, wherein the malignant tumor is a liver cancer; and the aforementioned method, wherein the RAR- α agonist is 4-[3,5-bis(trimethylsilyl)benzamido]benzoic acid. The present invention also provides the aforementioned method, which comprises the step of measuring a blood marker of which level changes in relation to the balance between the expression level of the class of the p160 family molecule and the expression level of the class of the SP110 family molecule.

From another aspect of the present invention, there is provided a kit comprising a combination of reagents for measuring the expression level of the class of the p160 family molecule and the expression level of the class of the SP110 family molecule in a sample collected from a malignant tumor of a patient.
From further aspects of the present invention, there are provided a method for therapeutic treatment of malignant tumor, which comprises:
(a) the step of collecting a sample of a malignant tumor from a patient with the malignant tumor,
(b) measuring an expression level of the class of the p160 family molecule and an expression level of the class of the SP110 family molecule in the sample, and
(c) observing a balance between the expression level of the class of the p160 family molecule and the expression level of the class of the SP110 family molecule, and administering an RAR- α agonist to the patient when the class of the p160 family molecule is dominant; and a medicament for therapeutic treatment of a malignant tumor, which comprises an RAR- α agonist as an active ingredient and is administered when the class of the p160 family molecule is dominant in a balance between an expression level of the class of the p160 family molecule and an expression level of the class of the SP110 family molecule in a patient.

### Brief Description of the Drawings

[Fig. 1] Fig. 1 shows results of observing expression of AIB1, one of the class of the p160 family molecules, in an established human derived hepatocellular carcinoma cell lines.
[Fig. 2] Fig. 2 shows results of observing expression of SRC-1, one of the class of the p160 family molecules, in an established human derived hepatocellular carcinoma cell lines.
[Fig. 3] Fig. 3 shows results of observing expression of TIF2, one of the class of the p160 family molecules, in an established human derived hepatocellular carcinoma cell lines.
[Fig. 4] Fig. 4 shows results of observing expression of SP110b, one of the class of the SP110 family molecules, in an established human derived hepatocellular carcinoma cell lines.

### Best Mode for Carrying out the Invention

The method of the present invention is a method for predicting therapeutic effect of an RAR- α agonist on malignant tumor, and characterized by measuring the expression level of the class of the p160 family molecule as a coactivator, that enhances activation of RAR- α transcription, and the expression level of the class of the SP110 family molecule as a corepressor, that inhibits activation of RAR- α transcription in malignant tumor of a patient, and observing a balance between these expression levels. Further, the method of the present invention is characterized by comprising the steps of measuring the expression level of the class of the p160 family molecule and the expression level of the class of the SP110 family molecule in a malignant tumor of a patient, observing a balance between the expression level of the class of the p160 family molecule and the expression level of the class of the SP110 family molecule, and determining that an RAR- α agonist is effective for therapeutic treatment of the malignant tumor in the patient when the class of the p160 family molecules is dominant.

The therapeutic effect of an RAR- α agonist referred to in the present invention include effects of cytoreducing and disappearing a tumor or stabilizing a tumor in a primary tumor, as well as effects of preventing of recurrence, metastasis and relapse of the tumor.
Examples of the class of the p160 family molecules include AIB1, SRC-1, TIF2, and the like. In the method of the present invention, expression level of AIB1 is preferably measured. It is known that AIB1 has an action as a steroid receptor coactivator and the like, and can be used for prediction of prognosis of hepatocellular carcinoma (Cancer, 95(11), pp.2346-2352, 2002). Therefore, the expression level of AIB1 can be easily measured by those skilled in the art. Further, it is also known that SRC-1 and TIF2 have an action as a steroid receptor coactivator and the like, and the expression levels of SRC-1 and TIF2 can be easily measured by those skilled in the art (International Patent Publication WO97/10337).
Examples of the class of the SP110 family molecules include SP110b and the like. It is known that SP110b is an RAR-α -selective corepressor (Hijikata W.k. et al., Mol. Endocrinol., 17(9), pp.1681-1692, 2003), and the expression level of SP110b can be easily measured by those skilled in the art.

In the specification, the expression of "observing a balance between the expression level of the class of the p160 family molecule and the expression level of the class of the SP110 family molecule" means, for example, to measure the expression levels of each class of the molecule in a sample and observe a ratio of these expression levels (coactivator/corepressor, i.e., the expression level of the class of the p160 family molecule/the expression level of the class of the SP110 family molecule). In the specification, the expression that "the class of the p160 family molecules is dominant" means, for example, that when the expression level of the class of the p160 family molecule and the expression level of the class of the SP110 family molecule are relatively compared, the expression level of the class of the p160 family molecule is observed so as to significantly increase the RAR- α transcription activity. For example, when a combination of AIB1 and SP110b is used, expression levels thereof in a sample collected from a malignant tumor of a patient are measured, and then a ratio of the expression levels (the expression level of AIB1/the expression level of SP110b) is calculated. When the ratio exceeds 0.05, preferably 0.1, it is determined that the class of the p160 family molecule is dominant, and an RAR- α agonist is effective for therapeutic treatment of the malignant tumor of the patient. When a combination of SRC-1 and SP110b is used, expression levels thereof in a sample collected from a malignant tumor of a patient are measured, and then a ratio of the expression levels (the expression level of SRC-1/the expression level of SP110b) is calculated. When the ratio exceeds 0.3, preferably 0.5, it is determined that the class of the p160 family molecule is dominant, and an RAR- α agonist is effective for therapeutic treatment of the malignant tumor of the patient. When a combination of TIF2 and SP110b is used, expression levels thereof in a sample collected from a malignant tumor of a patient are measured, and then a ratio of the expression levels (the expression level of TIF2/the expression level of SP110b) is calculated. When the ratio exceeds 0.1, preferably 0.12, it is determined that the class of the p160 family molecule is dominant, and an RAR- α agonist is effective for therapeutic treatment of the malignant tumor of the patient.

Type of the malignant tumor as an object of the determination by the method of the present invention is not particularly limited. Examples thereof include malignant tumors selected from the group consisting of solid cancers such as liver cancer, lung cancer, gastric cancer, colorectal cancer, pancreatic cancer, uterine cancer, ovarian cancer, breast cancer and prostate cancer, and blood cancers such as leukemia, lymphoma and myeloma. The object of determination is preferably liver cancer or lung cancer, more preferably hepatocellular carcinoma, most preferably advanced primary hepatocellular carcinoma. The method of the present invention can be applied to a patient before receiving cancer treatment by chemotherapy and/or radiation therapy or the like. Alternatively, the method can also be used as a determination method for implementing chemotherapy by using an RAR- α agonist as a secondary therapy for a patient having a tumor that acquires resistance to a primary therapy using chemotherapeutic agents other than RAR- α agonists and/or radiation therapy and failing to give expected response.

When the method of the present invention is performed, a biological sample is usually required to be extracted from a malignant tumor of a patient. Generally, a malignant tumor tissue is obtainable by biopsy, surgical operation, or the like. If the object is blood cancer, a sample may be collected by blood collection.
Examples of the means used in the present invention for measuring genes of the classes of the p160 family molecules and the SP110 family molecules or proteins as gene products thereof include RT-PCR described in the examples of the present invention as well as in-situ hybridization (Genome Research, 13, pp.1324-1334, 2003), immunohistologic staining (Journal of Pathology, 185, pp.25-31, 1998) using a tissue sample collected at the time of definite diagnosis and the like. However, the means are not limited to these examples so far that the means can achieve similar measurement.

Type of the RAR- α agonist of which efficacy can be determined by the method of the present invention is not particularly limited, and examples thereof include TAC-101 (4-[3,5-bis(trimethylsilyl)benzamido]benzoic acid, Yamakawa T., et al., J. Med. Chem., 33, pp.1430-1437, 1990), Am80 (4-[(5,6,7,8-tetrahydro-5,5,8,8-tetramethyl-2-naphthalenyl)carbamoyl]benzoic acid, Hashimoto Y., et al., Biochem. Biophys. Res. Commun., 166, pp.1300-1307, 1990), Am580 (4-[(5,6,7,8-tetrahydro-5,5,8,8-tetramethyl-2-naphthalenyl)carboxamidolbenzoic acid, Kagechika H., et al., J. Med. Chem., 31, pp.2182-2192, 1988), E6060 (4-{5-[7-fluoro-4-(trifluoromethyl)benzo[b]furan-2-yl]-1H-pyrrol-2-yl}benzoic acid, Yamauchi T., et al., J. Pharmacol. Exp. Ther., 312, pp.938-944, 2005), ER-38925 (4-(5-benzofuran-2-yl-1H-pyrrol-2-yl)benzoic acid, Yoshimura H., J. Med. Chem., 43, pp.2929-2937, 2000), and the like. However, the RAR- α agonists are not limited to these examples. According to the method of the present invention, efficacy of TAC-101 can be preferably determined. The binding affinity of a test agent to an RAR- α receptor subtype can be easily determined by, for example, a competition test for affinity of a test agent, radiolabeled with tritium or the like, to various receptor subtypes having a ligand binding region (Pijnappel W.W., et al., Nature (London), 366, pp.340-344, 1993; Apfel C., et al., Proc. Natl. Acad. Sci. U.S.A., 89(15), pp.7129-7133, 1992). For example, it is described that TAC-101 is an RAR- α -selective agonist in Hashimoto Y., et al., Biol. Pharm. Bull., 19(10), pp.1322-1328, 1996; Sun S.Y., et al., Cancer Res., 57(21), pp.4931-4939, 1997, and the like. In the aforementioned articles, TAC-101 is referred to as Am555S.

According to the method of the present invention, when the class of the p160 family molecule as a coactivator is dominant in the balance between expression level of the class of the p160 family molecule and that of the class of the SP110 family molecule, it can be determined that an RAR- α agonist is effective for therapeutic treatment of the malignant tumor in a patient. It is conventionally known that RAR- α agonists have an antitumor action. However, antitumor action correlating to the expression level of RAR- α has not been observed, and no method has been known by which whether or not an RAR- α agonist will effectively act on a specific patient as an antitumor agent can be reliably predicted before administration thereof.

By using the method of the present invention, it becomes possible to conveniently and accurately determine whether or not an RAR- α agonist will effectively act on a specific patient as an antitumor agent, and thus appropriate antitumor treatment becomes applicable. For the aforementioned purpose, the expression level of the class of the p160 family molecule and that of the class of the SP110 family molecule in a sample are measured, and their ratio (the class of the p160 family molecule/the class of the SP110 family molecule) may be calculated. Alternatively, for example, by using a blood marker of which level changes in relation to the ratio of the expression level of the class of the p160 family molecule and that of the class of the SP110 family molecule, a concentration of the marker in blood collected from a patient can be measured and used as a parameter as an alternative of the aforementioned ratio.

Further, the present invention also provides a method for therapeutic treatment of a malignant tumor. In this method for therapeutic treatment, a sample of a malignant tumor is collected from a patient with the malignant tumor, an expression level of the class of the p160 family molecule and an expression level of a corepressor in the sample are measured, and when the class of the p160 family molecule is dominant in the balance between the expression level of the class of the p160 family molecule and that of the class of the SP110 family molecule, an RAR- α agonist can be administered to the patient. Even if the class of the SP110 family molecules is dominant, an RAR- α agonist may be administered to the patient, however, complete response or partial response cannot be often expected.

For example, when liver cancer is treated with TAC-101 by applying the method of the present invention, TAC-101 is preferably administered to a patient with liver cancer at a dose of 10 to 30 mg/day, more preferably 15 to 25 mg/day, most preferably 20 mg/day. Further, therapeutic treatment is preferably performed by a treatment course consisting of everyday administration for 1 to 4 weeks at the aforementioned dose followed by rest for 1 to 3 weeks, or preferably performed by a treatment course consisting of everyday administration for 2 weeks followed by rest for 1 week. Repeating of either of the aforementioned treatment courses at least 4 times is also preferred. Although type of liver cancer is not particularly limited, primary liver cancer is preferred, and advanced primary hepatocellular carcinoma is more preferred. As a therapeutic treatment of liver cancer, a therapy selected from the group consisting of surgical hepatectomy, radiofrequency ablation, ethanol injection therapy and microwave coagulo-necrotic therapy may be performed in combination.

### Example

The present invention will be explained more specifically with reference to the example. However, the scope of the present invention is not limited to the following example.

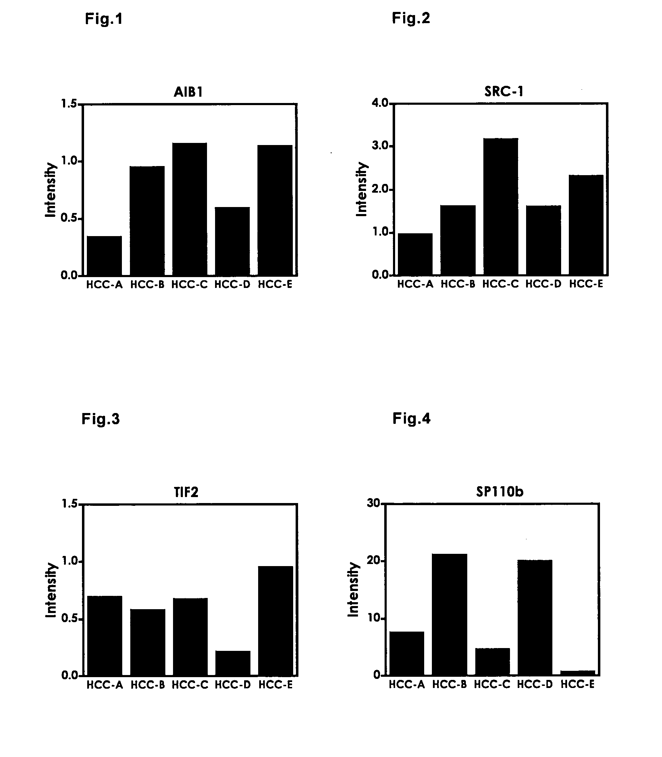

Figs. 1, 2 and 3 show results of observing expressions of AIB1, SRC-1, and TIF2, which are members of the p160 family molecules, in an established human derived hepatocellular carcinoma cell lines. Fig. 4 shows results of observing expression of SP 110b, which is the class of the SP110 family molecule, in an established human derived hepatocellular carcinoma cell lines. The test for observing the expression levels of the factors was performed as follows.
RNA was extracted from human hepatocellular carcinoma cells (RNeasy^{®} Mini Kit, QIAGEN) to prepare cDNA (2.1.5.1 Reverse Transcription System, Promega). Primers for PCR detection specific to various members of the p160 family molecules and the SP110 family molecules were prepared (Applied Biosystems Japan).

AIB1 primer sequences:
Forward: GCAGGCTGCATCCATCTATCA
Reverse: TGCCATTCATGTGCACCATAC
SRC-1 primer sequences:
Forward: GCAGAGAACCATCTTATGCCAGA
Reverse: GCTAAGCATTGTCCCATCATTCA
TIF2 primer sequences:
Forward: TGGCAAGAAGAGTTCCCATGA
Reverse: TTCTTACCAGGTCCTCCCAGC
SP110b primer sequences:
Forward: GTTGGATCCATGAGCACAAATCC
Reverse: GTTCTCGAGTCACCCGGGCTGAGCCC

Real-time PCR was performed by using the aforementioned cDNA. In a volume of 25 µL of cDNA diluted 25 times with distilled water, 0.4 µ M of primers for detection and 0.2 µ M of probe for detection (Universal PCR Master Mix) were applied in an automatic PCR product detection/assay system (ABI PRISM7700, Applied Biosystems Japan) to allow a gene amplification reaction. Values relative to an internal standard were calculated according to the following equation. Value relative to internal standard = (Value measured with probe and primers for detecting each factor to be measured)/(Value measured with TaqMan^{®} β-actin Control Reagents).

The test for confirming the RAR- α transcription activity was performed as follows.
Hepatocellular carcinoma cells of each type transfected with a reporter plasmid containing an RAR- α binding sequence with the SEAP gene in a downstream region (pRARE-SEAP-TA-vector, Clontech) were treated with an RAR- α agonist, and then the SEAP alkaline phosphatase activity in the culture supernatant was measured. The cultured hepatocellular carcinoma cells were transfected with the RARE reporter plasmid mixed in a transfection reagent prepared by using the culture broth and FuGENE6 solution at a ratio of 100:6 and cultured for 24 hours. The culture broth was replaced with the one containing TAC-101 as an RAR- α agonist, and the cells were cultured for further 24 hours. Then, the culture supernatant was collected, and transcription activity was confirmed by using an alkaline phosphatase activity measurement reagent (SEAP Detection Kit, Clontech). As for the transcription activity of RAR- α, the transcription activity of the treated cells was obtained as a relative value (T/C) based on that of untreated cells taken as 100, and a cancer cell line showing a response with a T/C of 200 or higher was determined to have a significantly high RAR- α transcription activity.

In Tables 1, 2 and 3, there are shown correlations between the ratios of the expression level of the class of the p160 family molecule and expression level of the class of the SP110 family molecule and the transcription activity of RAR- α in various human-derived hepatocellular carcinoma cell lines. It is shown by the results that higher ratios of the class of the p160 family molecule/the class of the SP110 family molecule, i.e., dominant of the class of the p160 family molecule, gave higher RAR- α transcription activity.

**[Table 1]**

| | Expression index | | Cofactor balance AIB1/SP110b | RAR response (T/C%) | | |
|---|---|---|---|---|---|---|
| | AIB1 | SP110b | | TAC-101 (µM) | | |
| | | | | 0.3 | 1 | 3 |
| HCC-A | 0.35 | 7.74 | **0.045** | 142 | 110 | 143 |
| HCC-B | 0.96 | 21.31 | **0.045** | 180 | 188 | 146 |
| **HCC-C** | 1.16 | 4.82 | **0.240** | **342** | **497** | **285** |
| HCC-D | 0.60 | 20.25 | **0.030** | 161 | 161 | 168 |
| **HCC-E** | 1.14 | 0.83 | **1.373** | **476** | **482** | **259** |

**[Table 2]**

| | Expression index | | Cofactor balance SRC-1/SP110b | RAR response (T/C%) | | |
|---|---|---|---|---|---|---|
| | SRC-1 | SP110b | | TAC-101 (µM) | | |
| | | | | 0.3 | 1 | 3 |
| HCC-A | 0.99 | 7.74 | **0.128** | 142 | 110 | 143 |
| HCC-B | 1.64 | 21.31 | **0.077** | 180 | 188 | 146 |
| **HCC-C** | 3.19 | 4.82 | **0.662** | **342** | **497** | **285** |
| HCC-D | 1.63 | 20.25 | **0.080** | 161 | 161 | 168 |
| **HCC-E** | 2.34 | 0.83 | **2.826** | **476** | **482** | **259** |

**[Table 3]**

| | Expression index | | Cofactor balance TIF2/SP110b | RAR response (T/C%) | | |
|---|---|---|---|---|---|---|
| | TIF2 | SP110b | | TAC-101 (uM) | | |
| | | | | 0.3 | 1 | 3 |
| HCC-A | 0.70 | 7.74 | 0.090 | 142 | 110 | 143 |
| HCC-B | 0.59 | 21.31 | 0.027 | 180 | 188 | 146 |
| HCC-C | 0.68 | 4.82 | 0.141 | **342** | **497** | **285** |
| HCC-D | 0.22 | 20.25 | 0.011 | 161 | 161 | 168 |
| HCC-E | 0.96 | 0.83 | 1.153 | **476** | **482** | **259** |

Further, as a result of studies of these cofactor balances and therapeutic effects, it was found that a high the RAR transcription activity induced by TAC-101 as an RAR- α agonist was achieved in the HCC-C and HCC-E cells, in which the class of the p160 family molecule was dominant, whereas the RAR transcription activity was weak in the HCC-D cells, in which SP110b was dominant.
An orthotopic hepatoma model was found to be successfully obtainable by transplanting these cell lines into the liver of a nude mouse. Accordingly, an experiment of therapeutic treatment was performed to examine susceptibility to RAR-α agonists. The cultured liver cancer cells were transplanted in an amount of 1 x 10⁶ cells into the outer left lobes of the livers in nude mice (BALB/cA Jcl-nu, Clea Japan, Inc.), TAC-101 as an RAR- α agonist was orally administered everyday as a 0.5% hydroxypropylmethylcellulose suspension (Shin-Etsu Chemical Co., Ltd.), and then the weights of tumors formed in the livers were measured 3 weeks later to obtain mean inhibition rates. The results obtained in these liver cancer models are shown in Table 4. Inhibitory effects of 81% and 67% against tumor growth were observed in the HCC-C and HCC-E models, respectively. Further, it was also observed by two-sided Student's t test that these antitumor effects were statistically significant inhibitory action. Whilst, the inhibitory rate was -3% in the HCC-D model, and no therapeutic effect was observed.

Further, correlation between the cofactor balance and survival advantage was examined by using cancer cell lines for which survival were evaluatable. When cells of the colorectal cancer cell line CAC, having an AIB1/SP110b ratio of 0.08 so as to be dominant in SP110b, and the aforementioned HCC-E cells are inoculated in the nude mouse spleen, dissemination easily occurs in the liver, resulting in formation of a metastatic condition in the liver. After 1 x 10⁶ of CAC or HCC-E cells were transplanted, and TAC-101 was orally administered every day for 4 weeks, the survival time of each model animal was observed. The results of survival advantage are shown in Table 5. In the CAC model, in which SP110b was dominant, prolongation of the survival time was only 26%, whereas in the HCC-E model, in which the class of the p160 family molecule was dominant, nearly twice longer survival time prolongation of 81% was confirmed. Further, this effect in the HCC-E model was also statistically significant (P < 0.001). These results clearly demonstrate that an RAR- α agonist is effective for cancer types in which the class of the p160 family molecule is dominant in the aforementioned ratio of the class of the p160 family molecule/the class of the SP110 family molecule expression.

**[Table 4]**

| Cell line | AIB1/SP110b | Inhibition (%) |
|---|---|---|
| HCC-C | 0.240 | 81 |
| HCC-D | 0.030 | -3 |
| HCC-E | 1.373 | 67 |

**[Table 5]**

| Cell line | AIB1/SP110b | Increase of life span (%) |
|---|---|---|
| CAC | 0.080 | 26 |
| HCC-E | 1.373 | 81 |

### Industrial Applicability

Therapeutic effect of an RAR- α agonist on malignant tumor such as liver cancer can be conveniently and accurately predicted by the method of the present invention.

## Claims

1. A method for predicting therapeutic effect of an RAR- α agonist on a malignant tumor, which comprises the steps of measuring an expression level of a class of p160 family molecule and an expression level of a class of SP110 family molecule in the malignant tumor of a patient and observing a balance between said expression levels.

2. The method according to claim 1, which comprises the steps of measuring the expression level of the class of the p160 family molecule and the expression level of the class of the SP110 family molecule in the malignant tumor of the patient, observing the balance between the expression level of the class of the p160 family molecule and the expression level of the class of the SP110 family molecule, and when the class of the p160 family molecule is dominant, determining that an RAR- α agonist is effective for therapeutic treatment of the malignant tumor of the patient.

3. The method according to claim 1 or 2, wherein the expression level of AIB 1, SRC-1 or TIF2 among the class of the p160 family molecules is measured.

4. The method according to claim 1 or 2, wherein the expression level of SP110b among the class of the SP110 family molecules is measured.

5. The method according to claim 1 or 2, wherein the expression levels of AIB1 and SP110b are measured in a sample collected from the malignant tumor of the patient, and when the ratio of the expression levels (the expression level of AIB 1/the expression level of SP110b) exceeds 0.05, it is determined that an RAR-α agonist is effective for therapeutic treatment of the malignant tumor of the patient.

6. The method according to claim 1 or 2, wherein the expression levels of SRC-1 and SP110b in the sample collected from the malignant tumor of the patient are measured, and when the ratio of the expression levels (the expression level of SRC-1/the expression level of SP110b) exceeds 0.3, it is determined that an RAR- α agonist is effective for therapeutic treatment of the malignant tumor of the patient.

7. The method according to claim 1 or 2, wherein the expression levels of TIF2 and SP110b in the sample collected from the malignant tumor of the patient are measured, and when the ratio of the expression levels (the expression level of TIF2/the expression level of SP110b) exceeds 0.1, it is determined that an RAR-α agonist is effective for therapeutic treatment of the malignant tumor of the patient.

8. The method according to any one of claims 1 to 7, wherein the malignant tumor is a malignant tumor selected from the group consisting of solid cancers of liver cancer, lung cancer, gastric cancer, colorectal cancer, pancreatic cancer, uterine cancer, ovarian cancer, breast cancer, and prostate cancer, and blood cancers of leukemia, lymphoma, and myeloma.

9. The method according to claim 8, wherein the malignant tumor is liver cancer.

10. The method according to claim 9, wherein the malignant tumor is hepatocellular carcinoma.

11. The method according to any one of claims 1 to 10, wherein the RAR· α agonist is 4-[3,5-bis(trimethylsilyl)benzamido]benzoic acid.

12. A method for therapeutic treatment of a malignant tumor, which comprises:
(a) the step of collecting a sample of a malignant tumor from a patient with the malignant tumor,
(b) measuring an expression level of a class of p160 family molecule and an expression level of a class of SP110 family molecule in the sample, and
(c) observing a balance between the expression level of the class of the p160 family molecule and the expression level of the class of the SP110 family molecule, and administering an RAR-α agonist to the patient when the class of the p160 family molecules is dominant.

13. A medicament for therapeutic treatment of malignant tumor, which comprises an RAR- α agonist as an active ingredient and is administered when a class of p160 family molecule is dominant in a balance between an expression level of the class of the p160 family molecule and an expression level of a class of SP110 family molecule in a patient.
